# EUROPEAN PATENT SPECIFICATION

(11) **EP 4 465 013 B1**
(45) Date of publication and mention of the grant of the patent: **10.12.2025**
(21) Application number: 23198615.9
(22) Date of filing: 20.09.2023
(51) Int. Cl.: G01N 1/31, G01N 1/38, G01N 1/40, G01N 1/44, G01N 35/00, G01N 35/10, G01N 35/04

(54) **AUTOMATIC SAMPLE PREPARATION SYSTEM**
AUTOMATISCHES PROBENVORBEREITUNGSSYSTEM
SYSTÈME AUTOMATIQUE DE PRÉPARATION D'ÉCHANTILLONS

(30) Priority: 17.05.2023 TW 112118274
(43) Date of publication of application: 20.11.2024
(73) Proprietor: Plexbio Co., Ltd., Taipei City, 11491 (TW)
(72) Inventor: Tsao, Dean, Taipei City (TW); LU, FENGKAN, Taipei City (TW); KAO, CHIEN TENG, Taipei City (TW)
(74) Representative: dompatent

(56) References cited:
- JP-A- 2012 161 340
- US-A1- 2015 337 400

## Description

### BACKGROUND

### 1. Technical Field

This disclosure relates to an automatic sample preparation system.

### 2. Related Art

In modern fields of disease diagnosis, drug development, environmental monitoring, food testing, criminal identification, and academic research, bioactive substances, such as cells, proteins, and genetic materials, are often required. Among them, genetic materials are one of the basic substances of life, including ribonucleic acid (RNA) and deoxyribonucleic acid (DNA), and widely exist in all animals, plants, microorganisms, and viruses. With the development of biotechnology, it is possible to extract genetic materials from organisms and utilize them in subsequent analysis and application. Equipment that is able to automatically extract genetic materials from samples has been developed. However, the forms of biological samples are quite diverse. Depending on different sampling methods, biological samples may be liquid, such as blood, saliva, and urine, solid paraffin, such as formalin-fixed paraffin-embedding (FFPE) samples, cultured cells, colonies, nasal swabs, etc. These various biologic samples must be pretreated before extraction so as to become available for automatic extraction equipment and then subjected to extraction. The extracted genetic materials often need to undergo an amplification reaction, such as a polymerase chain reaction (PCR). In addition, depending on subsequent analysis and application, the genetic materials sometimes need to be quantified before the amplification reaction.

However, the automatic extraction equipment currently on the market does not include a function of sample pretreatment nor include a function of subsequent quantification or solution preparation. In the process of sample pretreatment, quantification before amplification (pre-amplification quantification), or solution preparation, it requires a trained person and additional equipment to perform it. Manual operation not only consumes time and manpower but also generates human errors. More importantly, manual operation will greatly increase the risk of contamination.
US 2015/0037400 A1 discloses a system for controlling thermal cycler modules, the system processing samples, such as biological fluids, using assay cartridges which can be processed at different processing locations.
JP 2012/161340 A discloses a nucleic acid analyzer capable of conducting gene tests.

### SUMMARY

The present invention according to claim 1 provides an automatic sample preparation system, comprising: a sample pretreatment device configured to pretreat a sample to make the sample available for extraction; an extraction device configured to hold an extraction strip and utilize the extraction strip to extract a genetic material from the sample; a sample holder configured to hold a container for the sample, a container for the genetic material obtained from extraction, and an extraction tip; a pre-amplification quantification device configured to quantify the genetic material obtained from extraction; an amplification sample preparation device configured to prepare the genetic material obtained from extraction into a solution available for amplification reaction; a non-extraction tip holder configured to hold a non-extraction tip; and a pipetting device configured to move to the sample holder and pick up the extraction tip to mix or transfer liquid in the sample pretreatment device, the extraction device, and the sample holder, or configured to move to the non-extraction tip holder and pick up the non-extraction tip to mix or transfer liquid in the sample holder, the pre-amplification quantification device, and the amplification sample preparation device; wherein the extraction device and the sample holder are respectively disposed at opposite sides of the sample pretreatment device, and the pre-amplification quantification device, the amplification sample preparation device, and the non-extraction tip holder are disposed at a side of the sample holder away from the sample pretreatment device. The automatic sample preparation system further comprises a housing having an opening, wherein the sample pretreatment device, the extraction device, the sample holder, the pre-amplification quantification device, the amplification sample preparation device, the non-extraction tip holder, and the pipetting device are disposed inside the housing, and the pre-amplification quantification device, the amplification sample preparation device, and the non-extraction tip holder are between the sample holder and the opening.

### BRIEF DESCRIPTION OF THE DRAWINGS

The present disclosure will become more fully understood from the detailed description given hereinbelow and the accompanying drawings which are given by way of illustration only and thus are not limitative of the present disclosure and wherein:
FIG. 1 is a schematic view of an automatic sample preparation system according to an embodiment of the present disclosure;
FIG. 2 is one example of an extraction strip held at the extraction device;
FIG. 3A is a sample holder suitable for 10 mL of serum or plasma;
FIG. 3B is a sample holder suitable for paraffin, such as FFPE;
FIG. 3C is a sample holder suitable for virus or blood; and
FIG. 3D is a sample holder suitable for 5 mL of serum or plasma.

### DETAILED DESCRIPTION

In the following detailed description, for purposes of explanation, numerous specific details are set forth in order to provide a thorough understanding of the disclosed embodiments. According to the description, claims and the drawings disclosed in the specification, one skilled in the art may easily understand the concepts and features of the present invention. The following embodiments further illustrate various aspects of the present invention, but are not meant to limit the scope of the present invention.

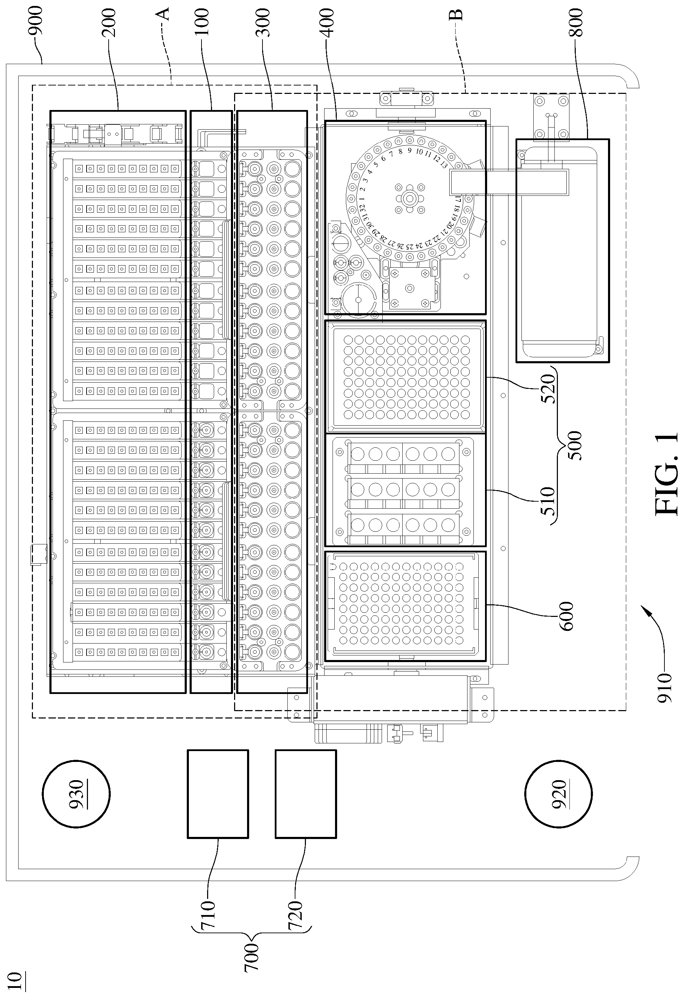

Please refer to FIG. 1, FIG. 1 is a schematic view of an automatic sample preparation system according to an embodiment of the present disclosure. According to an embodiment of the present disclosure, the automatic sample preparation system 10 includes a sample pretreatment device 100, an extraction device 200, a sample holder 300, a pre-amplification quantification device 400, an amplification sample preparation device 500, a non-extraction tip holder 600, and a pipetting device 700. The extraction device 200 and the sample holder 300 are respectively disposed at opposite sides of the sample pretreatment device 100. The pre-amplification quantification device 400, the amplification sample preparation device 500, and the non-extraction tip holder 600 are disposed at a side of the sample holder 300 away from the sample pretreatment device 100. Such an arrangement may reduce the distance for moving the pipetting device 700 among the aforementioned devices and holders, thereby reducing the risk of contamination or collision damage during movement.

The sample pretreatment device 100 is configured to pretreat a sample to make the sample available for extraction. Said term "available for extraction" refers to a liquid form that is able to be pipetted by a pipette and mixed with extraction reagents. The sample may be liquid or solid. The sample may include paraffin, blood, plasma, serum, saliva, virus, pathogen, bacteria, or cells. For example, when the sample needs to be diluted, the sample may be diluted in the sample pretreatment device 100 so as to become available for extraction. For example, the pretreatment device may include a dilution buffer supplier. The sample available for extraction may be transferred from the sample pretreatment device 100 to the extraction device 200 through the pipetting device 700, which will be described later, to be subjected to extraction.

The sample pretreatment device 100 may include a heating area configured to heat the sample placed here. For example, the heating area may have some heater. The heating area may encompass whole the sample pretreatment device 100 or part of the sample pretreatment device 100. For example, when the sample is paraffin, such as FFPE, the paraffin may be placed in the heating area of the sample pretreatment device 100 to be melted through heating and become available for extraction.

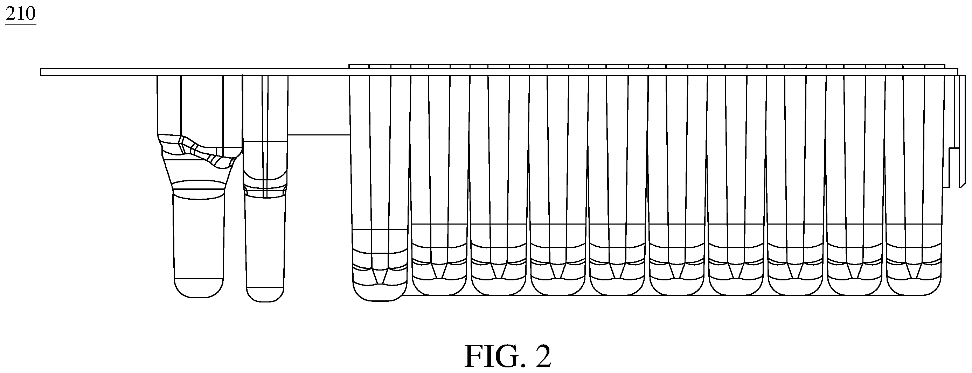

The extraction device 200 is configured to hold an extraction strip 210 and utilize the extraction strip 210 to extract a genetic material from the sample. For example, the extraction device 200 may include an extraction strip holder. FIG. 2 is one example of an extraction strip held at the extraction device. The extraction strip 210 may have a structure of several tubes connected to each other. Some tubes may contain reagents for extraction, such as magnetic beads-containing buffer, lysis buffer, wash buffer, and elution buffer. Other tubes may be empty tubes. The reagents in the extraction strip 210 may be varied depending on the different types of the sample. A part of the extraction strip 210 may be placed in the sample pretreatment device 100. For example, the extraction strip 210 as shown in FIG. 2 has two outmost empty tubes placed in the sample pretreatment device 100, and the two outmost empty tubes of the extraction strip 210 may be used to hold the sample to be pretreated. The extraction device 200 may have a foolproof structure. For example, the extraction device 200 may have a structure corresponding to the extraction strip 210 to position the extraction strip 210 so as to prevent incorrect operation of an extraction strip, thereby ensuring correct extraction process.

In the extraction device 200, liquid mixing and transferring may be performed by the pipetting device 700, which will be described later. In the extraction device 200 with the extraction strip 210 thereon, the extraction may be performed through binding the genetic material in the sample with the magnetic beads in the magnetic beads-containing buffer and transferring the genetic material to the next tube through pipetting the liquid in the tube by the pipetting device 700 so as to sequentially transfer the genetic material of the sample to each tube of the extraction strip 210. During extraction, liquid mixing may be performed through pipetting the liquid back and forth by the pipetting device 700. The extracted genetic material may be transferred to the sample holder 300 by the pipetting device 700.

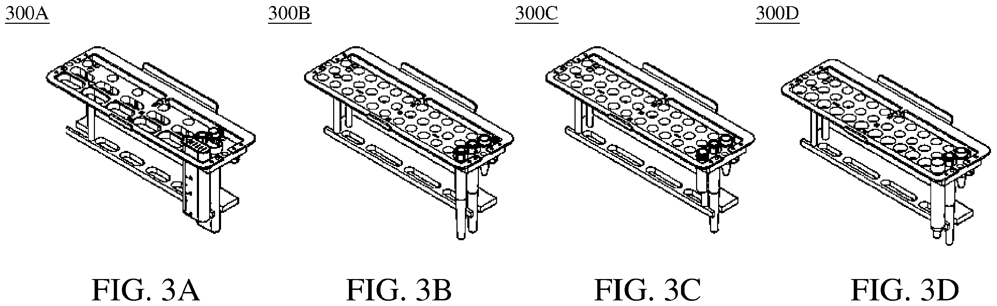

The sample holder 300 is configured to hold a container for the sample, a container for the genetic material obtained from extraction, and an extraction tip. The container for the sample may hold an original liquid sample that has not been extracted. The extraction tip may be picked up by the pipetting device 700, which will be described later, to be used for extraction. The sample holder 300 may sequentially hold the container for the sample, the extraction tip, and the container for the genetic material obtained from extraction in a direction toward the extraction device 200. In other words, the container for the genetic material obtained from extraction is closest to the extraction device 200. This allows the genetic material obtained from extraction to move into the container with the shortest distance, thereby reducing the risk of contamination during liquid transfer. The sample holder 300 may have various structures depending on the different types of the sample. For example, FIGs. 3A to 3D are sample holders 300 suitable for the different samples, FIG. 3A is a sample holder suitable 300A for 10 mL of serum or plasma, FIG. 3B is a sample holder 300B suitable for paraffin, such as FFPE, FIG. 3C is a sample holder 300C suitable for virus or blood, and FIG. 3D is a sample holder 300D suitable for 5 mL of serum or plasma. The sample holder 300 may have a foolproof structure. For example, the sample holder 300 may have pins or a structure corresponding to the container to position the sample holder 300 and the containers so as to prevent wrong installation of the sample holder 300 or wrong placement of the containers.

The pre-amplification quantification device 400 is configured to quantify the genetic material obtained from extraction. In the specific biochemical analysis or application, the genetic material in the sample sometimes needs to be quantified before an amplification reaction. The pre-amplification quantification device 400 may be an optical quantitation instrument, for example, a spectrometer. The genetic material obtained from extraction and hold in the sample holder 300 may be transferred to the pre-amplification quantification device 400 by the pipetting device 700, which will be described later, to be subjected to quantification. The pre-amplification quantification device 400 may be equipped with a LED light source, a filter, and an emitting diode, but the present disclosure is not limited thereto. For example, the pre-amplification quantification device 400 may have a 475 nm excitation filter and a 525 nm emission filter. The genetic material is measured at the specific wavelength and quantified by a calibration line established by built-in software.

The amplification sample preparation device 500 is configured to prepare the genetic material obtained from extraction into a solution available for amplification reaction. The amplification sample preparation device 500 may include an amplification reagent holder 510 and an amplification sample holder 520. The amplification reagent holder 510 may be configured to hold a reagent for amplification reaction, such as polymerase, dNTP, primer, and buffer. The amplification sample holder 520 may be configured to hold a prepared solution available for amplification reaction. The genetic material obtained from extraction and held in the sample holder 300 may be transferred to the amplification sample holder 520 by the pipetting device 700, which will be described later, and then the reagent in the amplification reagent holder 510 may be added to the genetic material obtained from extraction by the pipetting device 700 so as to prepare the solution available for amplification reaction. The prepared solution may be taken out from the automatic sample preparation system 10 to perform the subsequent amplification reaction or to be stored.

The non-extraction tip holder 600 is configured to hold a non-extraction tip, typically, a plurality of non-extraction tips. The non-extraction tip may be used for liquid mixing or transferring that is unrelated to extraction. In other words, the extraction tip held in the sample holder 300 may be used for liquid mixing or transferring in the sample pretreatment device 100, the extraction device 200, and the sample holder 300, while the non-extraction tip held in the non-extraction tip holder 600 may be used for liquid mixing or transferring in the sample holder 300, the pre-amplification quantification device 400, and the amplification sample preparation device 500. Using the extraction tip and the non-extraction tip separately may reduce the distance for moving the pipetting device 700 in a single procedure, thereby reducing the risk of contamination during liquid transfer or tip pickup of the pipetting device 700.

The pipetting device 700 is configured to move to the sample holder 300 and pick up the extraction tip to mix or transfer liquid in the sample pretreatment device 100, the extraction device 200, and the sample holder 300, or configured to move to the non-extraction tip holder 600 and pick up the non-extraction tip to mix or transfer liquid in the sample holder 300, the pre-amplification quantification device 400, and the amplification sample preparation device 500. For example, the pipetting device 700 may be a robotic arm equipped with a pipette. The pipetting device 700 may mix liquid by pipetting the liquid back and forth. The pipetting device 700 may automatically detect or manually set the liquid volume and pipet the liquid back and forth depending on the liquid volume to achieve totally mixing of the liquid. Compared to mixing by stirring, mixing by pipetting the liquid back and forth depending on the liquid volume may make the liquid mixing more uniform and prevent the damage caused by the collision due to stirring.

The pipetting device 700 may include a first pipetting device 710 and a second pipetting device 720. The moving range A of the first pipetting device 710 may include the sample pretreatment device 100, the extraction device 200, and the sample holder 300. The first pipetting device 710 may move to the sample holder 300 and pick up the extraction tip to mix or transfer liquid in the sample pretreatment device 100, the extraction device 200, and the sample holder 300. The moving range B of the second pipetting device 720 may include the sample holder 300, the pre-amplification quantification device 400, the amplification sample preparation device 500, and the non-extraction tip holder 600. The second pipetting device 720 may move to the non-extraction tip holder 600 and pick up the non-extraction tip to mix or transfer liquid in the sample holder 300, the pre-amplification quantification device 400, and the amplification sample preparation device 500. The first pipetting device 710 and the second pipetting device 720 may move in different ranges respectively, and this may reduce the distance for moving a single pipetting device, thereby reducing the risk of contamination during liquid transfer or tip pickup.

The extraction device 200, the sample pretreatment device 100, and the sample holder 300 may be sequentially arranged in a first direction. The pre-amplification quantification device 400, the amplification sample holder 520, the amplification reagent holder 510, and the non-extraction tip holder 600 may be sequentially arranged in a second direction. The first direction may be perpendicular to the second direction. Through such an arrangement, it may reduce the distance for moving the pipetting device 700 to the non-extraction tip holder 600 to pick up the non-extraction tip held thereon, moving to the amplification reagent holder 510 to pipet the reagent, and then to the amplification sample holder 520 to add the reagent to the genetic material held thereon, thereby reducing the risk of contamination during liquid transfer or tip pickup of the pipetting device 700.

The automatic sample preparation system 10 may further include a waste collector 800. The waste collector 800 may be configured to collect the used tip or collect waste. For example, the waste collector 800 may be a container or box disposed at the outermost of the aforementioned devices and holders, and the waste collector 800 may be detachably disposed in the automatic sample preparation system 10. For example, the waste collector 800 may be disposed inside the housing 900, and the waste collector 800 may be disposed between the pre-amplification quantification device 400 and the opening 910. For example, the pre-amplification quantification device 400 may be disposed between the waste collector 800 and the sample holder 300

The automatic sample preparation system 10 further includes a housing 900 having an opening 910. The sample pretreatment device 100, the extraction device 200, the sample holder 300, the pre-amplification quantification device 400, the amplification sample preparation device 500, the non-extraction tip holder 600, and the pipetting device 700 are disposed inside the housing 900. The pre-amplification quantification device 400, the amplification sample preparation device 500, and the non-extraction tip holder 600 are between the sample holder 300 and the opening 910. The housing 900 may further include a door or plate to cover the opening 910. Such an arrangement may be easy to take out the prepared solution available for amplification reaction from the opening 910 and prevent the sample pretreatment device 100, the extraction device 200, or the sample holder 300 from being touched, thereby reducing the risk of contamination or damage due to touching.

The automatic sample preparation system 10 may further include an image recognizer 920. The image recognizer 920 may be disposed inside the housing 900. The image recognizer 920 may be configured to recognize a label or a tag on the container for the sample or the container for the genetic material. The label or the tag may be text, numbers, barcodes, or QR codes. For example, the image recognizer 920 may be a camera. For example, when the container for the sample is a blood collection tube, the image recognizer 920 may recognize the label on the blood collection tube and send the information to a computer for data collation and analysis. The image recognizer 920 may also recognize the placement and arrangement of consumables. For example, the image recognizer 920 may recognize the numbers, placement, and arrangement of the non-extraction tips in the non-extraction tip holder 600 so that the pipetting device 700 may move to the correct position to pick up a tip, or a warning signal may be issued when the tips are insufficient.

The automatic sample preparation system 10 may further include an ultraviolet light sterilizer 930. The ultraviolet light sterilizer 930 may be disposed inside the housing 900. The ultraviolet light sterilizer 930 may be configured to sterilize the sample pretreatment device 100, the extraction device 200, the sample holder 300, the pre-amplification quantification device 400, the amplification sample preparation device 500, the non-extraction tip holder 600, and the pipetting device 700. The ultraviolet light sterilizer 930 may include an ultraviolet light emitting device, such as an ultraviolet light lamp, which may be disposed on the top inside the housing 900 or at any position that allows the ultraviolet light to irradiate to each of the aforementioned devices and holders. The ultraviolet light sterilizer 930 may ensure the inside of the housing 900 is a sterile environment when the sample is put in.

The following describes an example of a sample from put in the automatic sample preparation system 10 to taken out. First, in the case that the sample is liquid, the container containing the sample is placed in the sample holder 300, and then the sample is moved to the sample pretreatment device 100 by the first pipetting device 710 to be diluted or heated so as to become available for extraction. In the case that the sample is solid, the sample is directly placed in the tube of the extraction strip 210 which is in the sample pretreatment device 100 to be diluted or heated so as to become available for extraction. Then, the sample to be extracted placed in the sample pretreatment device 100 is moved to the extraction device 200 by the first pipetting device 710 and sequentially mixed with each reagent in the extraction strip 210 to perform extraction. The extracted sample is moved to the container held in the sample holder 300 by the first pipetting device 710. In the above process, liquid mixing or transferring is performed by the first pipetting device 710 with the extraction tip which is previously held in the sample holder 300.

Then, when the pre-amplification quantification is required, the extracted sample placed in the sample holder 300 is moved to the pre-amplification quantification device 400 by the second pipetting device 720 for quantification. Then, depending on the result of quantification, or in the case that the pre-amplification quantification is not required, the extracted sample placed in the sample holder 300 is moved to the amplification sample holder 520 of the amplification sample preparation device 500 by the second pipetting device 720. The reagents held in the amplification reagent holder 510 of the amplification sample preparation device 500 are added to the extracted sample by the second pipetting device 720 to prepare the solution available for amplification reaction. The prepared solution is taken out from the opening 910 to perform the subsequent amplification reaction or to be stored. In the above process, liquid mixing or transferring is performed by the second pipetting device 720 with the non-extraction tip which is previously held in the non-extraction tip holder 600.

The automatic sample preparation system according to an embodiment of the present disclosure integrates sample pretreatment, extraction, quantification before amplification (pre-amplification quantification), and amplification solution preparation into one system. The automatic sample preparation system may process not only liquid samples but also solid samples, such as paraffin. The pipetting device of the automatic sample preparation system may achieve totally mixing of the liquid by pipetting the liquid back and forth. Compared to mixing by stirring, mixing by pipetting the liquid back and forth may make the liquid mixing more uniform and prevent the damage caused by the collision due to stirring. The arrangement of each component in the automatic sample preparation system may reduce the distance for moving the pipetting device among the components, thereby reducing the risk of contamination or collision damage during movement. Making each procedure automated to replace manual operations may simplify operation, reduce time and labor costs, and increase efficiency, and the standardized procedures may reduce human errors, increase reliability, and more importantly, greatly reduce contamination risk.

## Claims

1. An automatic sample preparation system (10), comprising:
a sample pretreatment device (100) configured to pretreat a sample to make the sample available for extraction;
an extraction device (200) configured to hold an extraction strip (210) and utilize the extraction strip to extract a genetic material from the sample;
a sample holder (300) configured to hold a container for the sample, a container for the genetic material obtained from extraction, and an extraction tip;
a pre-amplification quantification device (400) configured to quantify the genetic material obtained from extraction;
an amplification sample preparation device (500) configured to prepare the genetic material obtained from extraction into a solution available for amplification reaction;
a non-extraction tip holder (600) configured to hold a non-extraction tip;
a pipetting device (700) configured to move to the sample holder (300) and pick up the extraction tip to mix or transfer liquid in the sample pretreatment device (100), the extraction device (200), and the sample holder (300), or configured to move to the non-extraction tip holder (600) and pick up the non-extraction tip to mix or transfer liquid in the sample holder (300), the pre-amplification quantification device (400), and the amplification sample preparation device (500); and
a housing (900) having an opening (910);
wherein the extraction device (200) and the sample holder (300) are respectively disposed at opposite sides of the sample pretreatment device (100), and the pre-amplification quantification device (400), the amplification sample preparation device (500), and the non-extraction tip holder (600) are disposed at a side of the sample holder (300) away from the sample pretreatment device (100),
wherein the sample pretreatment device (100), the extraction device (200), the sample holder (300), the pre-amplification quantification device (400), the amplification sample preparation device (500), the non-extraction tip holder (600), and the pipetting device (700) are disposed inside the housing (900), and
wherein the pre-amplification quantification device (400), the amplification sample preparation device (500), and the non-extraction tip holder (600) are between the sample holder (300) and the opening (910).

2. The automatic sample preparation system (10) of claim 1, further comprising a waste collector (800), wherein the waste collector (800) is disposed inside the housing (900), and the waste collector (800) is disposed between the pre-amplification quantification device (400) and the opening (910).

3. The automatic sample preparation system (10) of claim 1, wherein the amplification sample preparation device (500) comprises an amplification reagent holder (510) and an amplification sample holder (520), the amplification reagent holder (510) is configured to hold a reagent for amplification reaction, and the amplification sample holder (520) is configured to hold a prepared solution available for amplification reaction.

4. The automatic sample preparation system (10) of claim 3, wherein the extraction device (200), the sample pretreatment device (100), and the sample holder (300) are sequentially arranged in a first direction, the pre-amplification quantification device (400), the amplification sample holder (520), the amplification reagent holder (510), and the non-extraction tip holder (600) are sequentially arranged in a second direction, and the first direction is perpendicular to the second direction.

5. The automatic sample preparation system (10) of claim 1, further comprising a waste collector (800), wherein the pre-amplification quantification device (400) is disposed between the waste collector (800) and the sample holder (300).

6. The automatic sample preparation system (10) of claim 1, wherein the sample pretreatment device (100) comprises a heating area, and the heating area is configured to heat the sample.

7. The automatic sample preparation system of claim 1, wherein the pipetting device (700) mixes the liquid by pipetting the liquid back and forth.

8. The automatic sample preparation system of claim 1, wherein the pipetting device (700) comprises a first pipetting device (710) and a second pipetting device (720), the moving range of the first pipetting device (710) comprises the sample pretreatment device (100), the extraction device (200), and the sample holder (300), and the moving range of the second pipetting device (720) comprises the sample holder (300), the pre-amplification quantification device (400), the amplification sample preparation device (500), and the non-extraction tip holder (600).

9. The automatic sample preparation system (10) of claim 1, further comprising an image recognizer (920) configured to recognize a label or a tag on the container for the sample or the container for the genetic material.

10. The automatic sample preparation system (10) of claim 1, further comprising an ultraviolet light sterilizer (930) configured to sterilize the sample pretreatment device (100), the extraction device (200), the sample holder (300), the pre-amplification quantification device (400), the amplification sample preparation device (500), the non-extraction tip holder (600), and the pipetting device (700).

## Patentansprüche

1. Automatisches Probenvorbereitungssystem (10), das aufweist:
eine Probenvorbehandlungsvorrichtung (100), die so ausgebildet ist, dass sie eine Probe vorbehandelt, um die Probe für die Extraktion verfügbar zu machen;
eine Extraktionsvorrichtung (200), die so ausgebildet ist, dass sie einen Extraktionsstreifen (210) hält und den Extraktionsstreifen verwendet, um ein genetisches Material aus der Probe zu extrahieren;
einen Probenhalter (300), der so ausgebildet ist, dass er einen Behälter für die Probe, einen Behälter für das bei der Extraktion erhaltene genetische Material und eine Extraktionsspitze hält;
eine Voramplifikationsquantifizierungsvorrichtung (400), die so ausgebildet ist, dass sie das aus der Extraktion erhaltene genetische Material quantifiziert;
eine Amplifikationsprobenvorbereitungsvorrichtung (500), die so ausgebildet ist, dass sie das durch Extraktion erhaltene genetische Material in eine für die Amplifikationsreaktion verfügbare Lösung vorbereitet;
einen Nicht-Extraktionsspitzenhalter (600), der so ausgebildet ist, dass er eine Nicht-Extraktionsspitze hält;
eine Pipettiervorrichtung (700), die so ausgebildet ist, dass sie sich zu dem Probenhalter (300) bewegt und die Extraktionsspitze aufnimmt, um Flüssigkeit in der Probenvorbehandlungsvorrichtung (100), der Extraktionsvorrichtung (200) und dem Probenhalter (300) zu mischen oder zu transferieren, oder so ausgebildet ist, dass sie sich zu dem Nicht-Extraktionsspitzenhalter (600) bewegt und die Nicht-Extraktionsspitze aufnimmt, um Flüssigkeit in dem Probenhalter (300), der Voramplifikationsquantifizierungsvorrichtung (400) und der Amplifikationsprobenvorbereitungsvorrichtung (500) zu mischen oder zu transferieren; und
ein Gehäuse (900) mit einer Öffnung (910);
wobei die Extraktionsvorrichtung (200) und der Probenhalter (300) jeweils an entgegengesetzten Seiten der Probenvorbehandlungsvorrichtung (100) angeordnet sind und die Voramplifikationsquantifizierungsvorrichtung (400), die Amplifikationsprobenvorbereitungsvorrichtung (500) und der Nicht-Extraktionsspitzenhalter (600) an einer Seite des Probenhalters (300) entfernt von der Probenvorbehandlungsvorrichtung (100) angeordnet sind,
wobei die Probenvorbehandlungsvorrichtung (100), die Extraktionsvorrichtung (200), der Probenhalter (300), die Voramplifikationsquantifizierungsvorrichtung (400), die Amplifikationsprobenvorbereitungsvorrichtung (500), der Nicht-Extraktionsspitzenhalter (600) und die Pipettiervorrichtung (700) im Inneren des Gehäuses (900) angeordnet sind, und
wobei die Voramplifikationsquantifizierungsvorrichtung (400), die Amplifikationsprobenvorbereitungsvorrichtung (500), und der Nicht-Extraktionsspitzenhalter (600) zwischen dem Probenhalter (300) und der Öffnung (910) liegen.

2. Automatisches Probenvorbereitungssystem (10) nach Anspruch 1, das ferner einen Abfallsammler (800) aufweist, wobei der Abfallsammler (800) im Inneren des Gehäuses (900) angeordnet ist und der Abfallsammler (800) zwischen der Voramplifikationsquantifizierungsvorrichtung (400) und der Öffnung (910) angeordnet ist.

3. Automatisches Probenvorbereitungssystem (10) nach Anspruch 1, wobei die Amplifikationsprobenvorbereitungsvorrichtung (500) einen Amplifikationsreagenzhalter (510) und einen Amplifikationsprobenhalter (520) aufweist, wobei der Amplifikationsreagenzhalter (510) so ausgebildet ist, dass er ein Reagenz für eine Amplifikationsreaktion hält, und der Amplifikationsprobenhalter (520) so ausgebildet ist, dass er eine vorbereitete Lösung hält, die für eine Amplifikationsreaktion verfügbar ist.

4. Automatisches Probenvorbereitungssystem (10) nach Anspruch 3, wobei die Extraktionsvorrichtung (200), die Probenvorbehandlungsvorrichtung (100) und der Probenhalter (300) aufeinanderfolgend in einer ersten Richtung angeordnet sind, die Voramplifikationsquantifizierungsvorrichtung (400), der Amplifikationsprobenhalter (520), der Amplifikationsreagenzhalter (510) und der Nicht-Extraktionsspitzenhalter (600) aufeinanderfolgend in einer zweiten Richtung angeordnet sind und die erste Richtung senkrecht zur zweiten Richtung verläuft.

5. Automatisches Probenvorbereitungssystem (10) nach Anspruch 1, das ferner einen Abfallsammler (800) aufweist, wobei die Voramplifikationsquantifizierungsvorrichtung (400) zwischen dem Abfallsammler (800) und dem Probenhalter (300) angeordnet ist.

6. Automatisches Probenvorbereitungssystem (10) nach Anspruch 1, wobei die Probenvorbehandlungsvorrichtung (100) einen Heizbereich aufweist und der Heizbereich zum Erhitzen der Probe ausgebildet ist.

7. Automatisches Probenvorbereitungssystem nach Anspruch 1, wobei die Pipettiervorrichtung (700) die Flüssigkeit durch Hin- und Herpipettieren der Flüssigkeit mischt.

8. Automatisches Probenvorbereitungssystem nach Anspruch 1, wobei die Pipettiervorrichtung (700) eine erste Pipettiervorrichtung (710) und eine zweite Pipettiervorrichtung (720) umfasst, der Bewegungsbereich der ersten Pipettiervorrichtung (710) die Probenvorbehandlungsvorrichtung (100), die Extraktionsvorrichtung (200) und den Probenhalter (300) umfasst, und der Bewegungsbereich der zweiten Pipettiervorrichtung (720) den Probenhalter (300), die Voramplifikationsquantifizierungsvorrichtung (400), die Amplifikationsprobenvorbereitungsvorrichtung (500) und den Nicht-Extraktionsspitzenhalter (600) umfasst.

9. Automatisches Probenvorbereitungssystem (10) nach Anspruch 1, das ferner einen Bilderkenner (920) aufweist, der so ausgebildet ist, dass er ein Etikett oder ein Schild auf dem Behälter für die Probe oder dem Behälter für das genetische Material erkennt.

10. Automatisches Probenvorbereitungssystem (10) nach Anspruch 1, das ferner einen Ultraviolettlicht-Sterilisator (930) aufweist, der so ausgebildet ist, dass er die Probenvorbehandlungsvorrichtung (100), die Extraktionsvorrichtung (200), den Probenhalter (300), die Voramplifikationsquantifizierungsvorrichtung (400), die Amplifikationsprobenvorbereitungsvorrichtung (500), den Nicht-Extraktionsspitzenhalter (600) und die Pipettiervorrichtung (700) sterilisiert.

## Revendications

1. Système automatique de préparation d'échantillon (10), comprenant :
un dispositif de prétraitement d'échantillon (100) configuré pour prétraiter un échantillon afin de rendre l'échantillon disponible pour une extraction ;
un dispositif d'extraction (200) configuré pour maintenir une bande d'extraction (210) et utiliser la bande d'extraction pour extraire un matériel génétique de l'échantillon ;
un porte-échantillon (300) configuré pour maintenir un contenant pour l'échantillon, un contenant pour le matériel génétique obtenu à partir de l'extraction, et une pointe d'extraction ;
un dispositif de quantification de pré-amplification (400) configuré pour quantifier le matériel génétique obtenu par l'extraction ;
un dispositif de préparation d'échantillon d'amplification (500) configuré pour préparer le matériel génétique obtenu par l'extraction dans une solution disponible pour une réaction d'amplification ;
un support de pointe de non-extraction (600) configuré pour supporter une pointe de non-extraction ;
un dispositif de pipetage (700) configuré pour se déplacer vers le porte-échantillon (300) et prendre la pointe d'extraction pour mélanger ou transférer du liquide dans le dispositif de prétraitement d'échantillon (100), le dispositif d'extraction (200) et le porte-échantillon (300), ou configuré pour se déplacer vers le support de pointe de non-extraction (600) et prendre la pointe de non-extraction pour mélanger ou transférer du liquide dans le porte-échantillon (300), le dispositif de quantification de pré-amplification (400) et le dispositif de préparation d'échantillon d'amplification (500) ; et
un boîtier (900) présentant une ouverture (910) ;
dans lequel le dispositif d'extraction (200) et le porte-échantillon (300) sont disposés respectivement sur des côtés opposés du dispositif de prétraitement d'échantillon (100), et le dispositif de quantification de pré-amplification (400), le dispositif de préparation d'échantillon d'amplification (500) et le support de pointe de non-extraction (600) sont disposés sur un côté du porte-échantillon (300) loin du dispositif de prétraitement d'échantillon (100),
dans lequel le dispositif de prétraitement d'échantillon (100), le dispositif d'extraction (200), le porte-échantillon (300), le dispositif de quantification de pré-amplification (400), le dispositif de préparation d'échantillon d'amplification (500), le support de pointe de non-extraction (600) et le dispositif de pipetage (700) sont disposés à l'intérieur du boîtier (900), et
dans lequel le dispositif de quantification de pré-amplification (400), le dispositif de préparation d'échantillon d'amplification (500) et le support de pointe de non-extraction (600) se trouvent entre le porte-échantillon (300) et l'ouverture (910).

2. Système automatique de préparation d'échantillon (10) de la revendication 1, comprenant en outre un collecteur de déchets (800), dans lequel le collecteur de déchets (800) est disposé à l'intérieur du boîtier (900), et le collecteur de déchets (800) est disposé entre le dispositif de quantification de pré-amplification (400) et l'ouverture (910).

3. Système automatique de préparation d'échantillon (10) de la revendication 1, dans lequel le dispositif de préparation d'échantillon d'amplification (500) comprend un porte-réactif d'amplification (510) et un porte-échantillon d'amplification (520), le porte-réactif d'amplification (510) est configuré pour contenir un réactif pour une réaction d'amplification, et le porte-échantillon d'amplification (520) est configuré pour contenir une solution préparée disponible pour une réaction d'amplification.

4. Système automatique de préparation d'échantillon (10) de la revendication 3, dans lequel le dispositif d'extraction (200), le dispositif de prétraitement d'échantillon (100) et le porte-échantillon (300) sont agencés séquentiellement dans une première direction, le dispositif de quantification de pré-amplification (400), le porte-échantillon d'amplification (520), le porte-réactif d'amplification (510) et le support de pointe de non-extraction (600) sont agencés séquentiellement dans une seconde direction, et la première direction est perpendiculaire à la seconde direction.

5. Système automatique de préparation d'échantillon (10) de la revendication 1, comprenant en outre un collecteur de déchets (800), dans lequel le dispositif de quantification de pré-amplification (400) est disposé entre le collecteur de déchets (800) et le porte-échantillon (300).

6. Système automatique de préparation d'échantillon (10) de la revendication 1, dans lequel le dispositif de prétraitement d'échantillon (100) comprend une zone de chauffage, et la zone de chauffage est configurée pour chauffer l'échantillon.

7. Système automatique de préparation d'échantillon de la revendication 1, dans lequel le dispositif de pipetage (700) mélange le liquide en renouvelant le pipetage du liquide par aspiration-distribution.

8. Système automatique de préparation d'échantillon de la revendication 1, dans lequel le dispositif de pipetage (700) comprend un premier dispositif de pipetage (710) et un second dispositif de pipetage (720), la plage de déplacement du premier dispositif de pipetage (710) comprend le dispositif de prétraitement d'échantillon (100), le dispositif d'extraction (200) et le porte-échantillon (300), et la plage de déplacement du second dispositif de pipetage (720) comprend le porte-échantillon (300), le dispositif de quantification de pré-amplification (400), le dispositif de préparation d'échantillon d'amplification (500) et le support de pointe de non-extraction (600).

9. Système automatique de préparation d'échantillon (10) de la revendication 1, comprenant en outre un dispositif de reconnaissance d'image (920) configuré pour reconnaître une étiquette ou un marqueur sur le contenant pour l'échantillon ou le contenant pour le matériel génétique.

10. Système automatique de préparation d'échantillon (10) de la revendication 1, comprenant en outre un stérilisateur à lumière ultraviolette (930) configuré pour stériliser le dispositif de prétraitement d'échantillon (100), le dispositif d'extraction (200), le porte-échantillon (300), le dispositif de quantification de pré-amplification (400), le dispositif de préparation d'échantillon d'amplification (500), le support de pointe de non-extraction (600) et le dispositif de pipetage (700).
